# EUROPEAN PATENT SPECIFICATION

(11) **EP 4 019 855 B1**
(45) Date of publication and mention of the grant of the patent: **25.10.2023**
(21) Application number: 20873186.9
(22) Date of filing: 10.09.2020
(51) Int. Cl.: F24F 11/39, F24F 11/48, F24F 11/52, F24F 8/90, F24F 11/65, F24F 1/0076, F24F 1/0071, F24F 8/22

(54) **AIR CONDITIONER**
KLIMAANLAGE
CLIMATISEUR

(30) Priority: 30.09.2019 JP 2019180012
(43) Date of publication of application: 29.06.2022
(73) Proprietor: Daikin Industries, Ltd., Osaka-shi, Osaka 530-0001 (JP)
(72) Inventor: NUNO, Hayato, Osaka 530-8323 (JP); ITOU, Hiroshi, Osaka 530-8323 (JP)
(74) Representative: Hoffmann Eitle
(86) International application number: PCT/JP2020/034252
(87) International publication number: WO 2021/065402

(56) References cited:
- CN-A- 109 210 631
- JP-A- 2001 324 195
- JP-A- 2001 324 195
- JP-A- 2007 205 617
- JP-A- 2009 133 498
- JP-A- 2009 133 498
- JP-A- 2010 179 798
- JP-A- 2012 149 855
- JP-A- 2016 125 710
- JP-A- 2016 125 710
- JP-A- 2018 189 351
- JP-A- 2018 200 127
- JP-A- 2018 200 127

## Description

The present invention relates to air conditioners.

### BACKGROUND ART

There is known an air conditioner in which a drain pan where drain water is stored is irradiated with deep ultraviolet rays having a relatively short wavelength among the ultraviolet rays (see, for example, JP 2017 133700 A). The irradiation with deep ultraviolet rays causes denaturation or inactivation of bacteria, mold, or the like contained in the drain water (hereinafter, referred to as "sterilization").

### SUMMARY OF INVENTIONTECHNICAL PROBLEMS

JP 2017 133700 A discloses that an irradiating operation is performed in relation to a cooling operation, but does not disclose how to control when a stop operation to stop the irradiating operation is performed during the irradiating operation. When the stop operation is performed when sterilization has not been completed yet, bacteria, mold, or the like remaining due to non-completion of sterilization easily propagates in drain water. In such a state, it is difficult to keep the inside of an indoor unit clean.

It is therefore an object of the present invention to provide an air conditioner capable of keeping the inside of an indoor unit clean.
CN 10 9210631 A discloses an air conditioner indoor unit with a sterilization function, a control method and an air conditioner. The air conditioner indoor unit with the sterilization function comprises a bottom shell; a negative ion generator which is arranged in the bottom shell and can release negative ions to air for sterilization and purification; and an ultraviolet lamp which is arranged in the bottom shell and can be used for ultraviolet radiation to the air for sterilization and purification. Through the air conditioner indoor unit, the negative ion generator and the ultraviolet lamp can be used for sterilization and purification of the air entering the indoor unit jointly, and the indoor air sterilization and the interior sterilization of the air conditioner are achieved; the sterilization effect is improved, and bacteria and microorganisms can be killed effectively; and the influence of bacteria breeding on user health is avoided.
JP 2001 324195 A discloses an air-conditioning device which comprises a heat exchanger in a casing situated in an attic space; a drain pan situated below the heat exchanger, and a lamp to effect irradiation with light containing ultraviolet rays. The drain pan can be sterilized by ultraviolet rays. The lamp can be lighted for a given time through operation of a lamp switch of a remote controller. Further, the lamp may be automatically lighted at intervals of a specified time and lighted during starting or a stop of an air- conditioning function. The heat exchanger and the drain pan may form a photocatalyst carrier and reflection members and may be situated. Thus, antibacterial operation is obtained during a longer period than antibacterial treatment.
JP 2016 125710 A discloses an air conditioner which is capable of performing a filter cleaning operation for cleaning a filter and comprises a remote controller carrying out a stop operation to stop the filter cleaning operation halfway, and the remote controller transmits a notification to attract user's attention to confirm whether to stop the filter cleaning operation when having carried out the stop operation.
JP 2018 200127 A discloses an air conditioner which includes one or more display lamps for indicating an operation state, and a control part having a drying mode for drying the inside of an indoor machine and a cleaning mode for cleaning an indoor heat exchanger. The control part lights the same display lamp (e.g., "operation" lamp or "clean" lamp) when starting operation or during the operation in the drying mode and in the cleaning mode, and directs a vertical wind direction plate in different directions in the drying mode and in the cleaning mode.

### SOLUTIONS TO PROBLEMS

An air conditioner according to the present invention is defined by claim 1. It includes: a control unit configured to control an air conditioning operation and control a maintenance operation to perform maintenance on an indoor unit of the air conditioner after the air conditioning operation is stopped; and an operation unit provided for performing a stop operation to stop the maintenance operation. The maintenance operation includes an irradiating operation to irradiate an irradiation area of the indoor unit with ultraviolet rays, and the control unit controls the irradiating operation to continue the irradiating operation even when the stop operation is performed by the operation unit.

The air conditioner according to the present invention causes the irradiating operation to continue even when the stop operation to stop the maintenance operation is performed, so that it is possible to keep the inside of the indoor unit clean. In particular, the maintenance operation includes a second maintenance operation including at least either an internal cleaning operation or a filter cleaning operation. The air conditioner can keep components built in the indoor unit clean. When the stop operation is performed, the control unit controls the second maintenance operation to stop the second maintenance operation. The air conditioner can reflect the intention of the user about the stop of the second maintenance operation by stopping the second maintenance operation in response to the stop operation to stop the second maintenance operation performed by the user.

Dependent claims relate to preferred embodiments. The air conditioner according to an embodiment further includes a notification unit configured to issue a notification about the irradiating operation. The control unit controls the notification unit to issue the notification about the continuation of the irradiating operation.

The air conditioner configured as described above can make the user aware of the continuation of the irradiating operation.

In the air conditioner according to an embodiment, when a forced operation is performed to forcibly stop the irradiating operation, the control unit controls the notification unit to issue the notification about the stop of the irradiating operation and controls the irradiating operation to forcibly stop the irradiating operation.

The air conditioner configured as described above can not only make the user aware of the stop of the irradiating operation but also reflect the intention of the user in the irradiating operation.

In the air conditioner according to an embodiment, the forced operation is an operation different from the stop operation to stop the maintenance operation.

The air conditioner configured as described above can prevent the user from stopping the irradiating operation because the forced operation is an operation different from the stop operation to stop the maintenance operation.

The air conditioner configured as described above can prevent the user from stopping the irradiating operation because the forced operation is complicated.

### BRIEF DESCRIPTION OF DRAWINGS

- Fig. 1 is a diagram illustrating a refrigerant circuit of an air conditioner according to an embodiment.
- Fig. 2 is a control block diagram of the air conditioner illustrated in Fig. 1 .
- Fig. 3 is a schematic cross-sectional view of an indoor unit that is out of operation, the indoor unit being a component of the air conditioner illustrated in Fig. 1 .
- Fig. 4 is a diagram illustrating a state where a panel of a remote control is closed.

Fig. 5 is a diagram illustrating a state where the panel of the remote control is opened.
Fig. 6 is a timing chart of a maintenance operation of the air conditioner.
Fig. 7 is a control flowchart of the maintenance operation of the air conditioner.

### DESCRIPTION OF EMBODIMENT

Hereinafter, an air conditioner according to an embodiment of the present invention will be described with reference to the drawings. Note that the same parts in the drawings are denoted by the same reference sign, and no redundant description will be given.

### [Overall configuration of air conditioner 1]

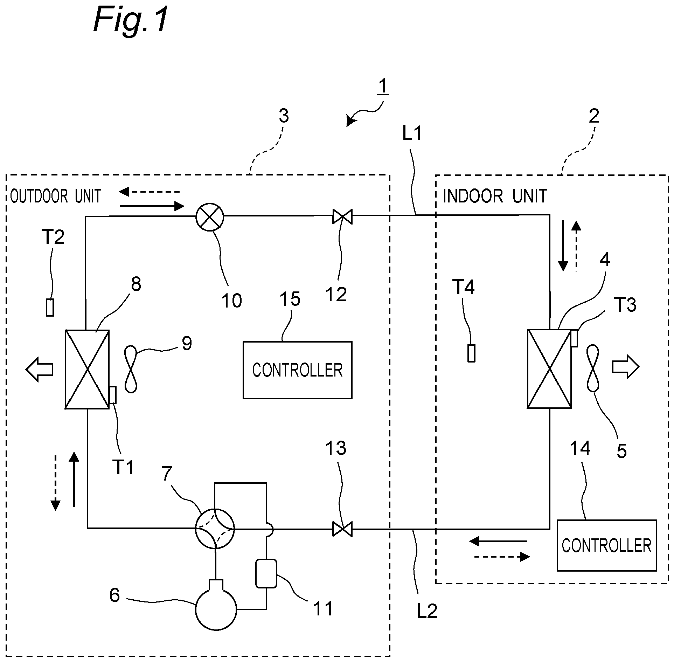

Fig. 1 is a diagram illustrating a refrigerant circuit of an air conditioner 1 according to the embodiment of the present invention. As illustrated in Fig. 1, the air conditioner 1 includes an indoor unit 2 installed indoors and an outdoor unit 3 installed outdoors, the indoor unit 2 and the outdoor unit 3 being connected with each other via connection pipes L1, L2. The air conditioner 1 is of a type in which the indoor unit 2 is paired one-to-one with the outdoor unit 3.

The indoor unit 2 is equipped with an indoor heat exchanger 4 and an indoor fan 5. The outdoor unit 3 is equipped with a compressor 6, a four-way switching valve 7, an outdoor heat exchanger 8, an outdoor fan 9, an electric expansion valve (hereinafter, referred to as an expansion valve) 10 as an example of the decompressing mechanism, and an accumulator 11. The outdoor unit 3 is further provided with a liquid-side shutoff valve 12 and a gas-side shutoff valve 13.

The compressor 6, the four-way switching valve 7, the outdoor heat exchanger 8, the expansion valve 10, the indoor heat exchanger 4, the accumulator 11, and the compressor 6 are connected in this order via a refrigerant pipe and the connection pipes L 1, L2 to form a refrigerant circuit. The liquid-side shutoff valve 12 is interposed between the expansion valve 10 and the connection pipe L1, and the gas-side shutoff valve 13 is interposed between the four-way switching valve 7 and the connection pipe L2.

In the refrigerant circuit, the compressor 6 has a discharge port connected to the outdoor heat exchanger 8 via the four-way switching valve 7 and has an intake port connected to the indoor heat exchanger 4 via the four-way switching valve 7 and the accumulator 11.

A remote controller 17 (hereinafter, referred to as a "remote control 17") can bring the air conditioner 1 configured as described above into cooling operation, dehumidifying operation, and heating operation (hereinafter, collectively referred to as a "normal operation"). The remote control 17 can switch, start, or stop various operations, set an indoor temperature, set a rotational speed of the indoor fan 5, and the like. The air conditioner 1 configured as described above allows the remote control 17 to enable to set a maintenance operation for keeping internal members built in the indoor unit 2 clean.

During the cooling operation and the dehumidifying operation, a cooling cycle is established as indicated by solid arrows in which a refrigerant discharged from the compressor 6 sequentially flows from the four-way switching valve 7 to the indoor heat exchanger 4 through the outdoor heat exchanger 8 and the expansion valve 10 and returns to the compressor 6 through the four-way switching valve 7 and the accumulator 11. That is, the outdoor heat exchanger 8 functions as a condenser, and the indoor heat exchanger 4 functions as an evaporator. Note that, during the dehumidifying operation, although the indoor fan 5 is driven to an extent less than during the cooling operation, the refrigerant passing through the indoor heat exchanger 4 evaporates as a result of exchanging heat with indoor air. This causes moisture in the air to be condensed and collected on a surface of the indoor heat exchanger 4, thereby dehumidifying the air inside the room. Therefore, an operation during which condensed water is generated on the surface of the indoor heat exchanger 4 such as the cooling operation and the dehumidifying operation is herein referred to as a cooling operation.

On the other hand, during the heating operation, a heating cycle is established as indicated by dashed arrows in which the four-way switching valve 7 is switched to cause the refrigerant discharged from the compressor 6 to sequentially flow from the four-way switching valve 7 to the outdoor heat exchanger 8 through the indoor heat exchanger 4 and the expansion valve 10 and return to the compressor 6 through the four-way switching valve 7 and the accumulator 11. That is, the indoor heat exchanger 4 functions as a condenser, and the outdoor heat exchanger 8 functions as an evaporator.

As illustrated in Fig. 1, the indoor unit 2 is equipped with an indoor-unit controller (control unit) 14 that controls various operations of the indoor unit 2, and the outdoor unit 3 is equipped with an outdoor-unit controller (control unit) 15 that controls various operations of the outdoor unit 3. The air conditioner 1 is controlled as a whole by the indoor-unit controller (control unit) 14 or the outdoor-unit controller (control unit) 15, or under cooperation between the indoor-unit controller (control unit) 14 and the outdoor-unit controller (control unit) 15. Therefore, at least either the indoor-unit controller 14 or the outdoor-unit controller 15 acts as a control unit 16 that controls various operations of the air conditioner 1.

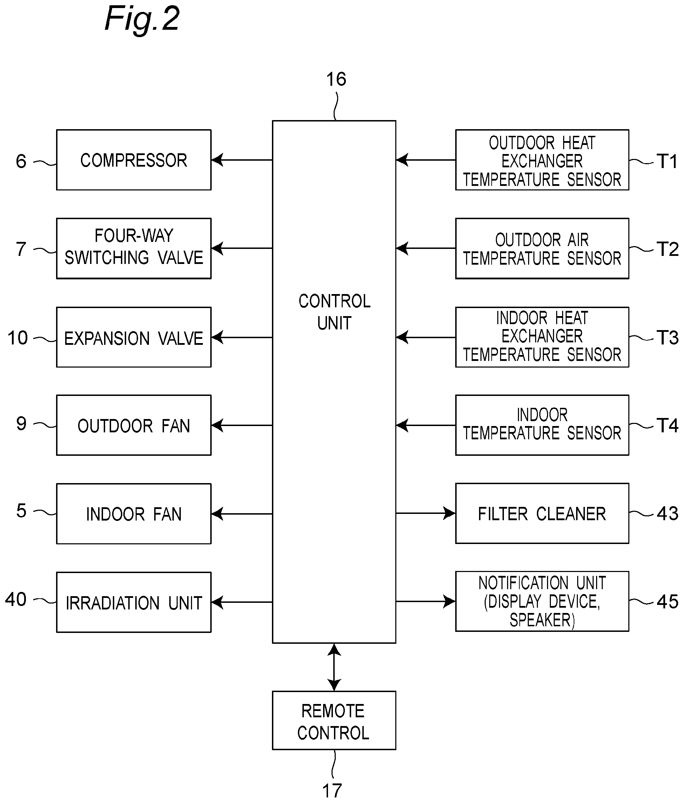

As illustrated in Fig. 2, the compressor 6, the four-way switching valve 7, the expansion valve 10, the indoor fan 5, and the outdoor fan 9 are connected to the control unit 16. Specifically, various drive units (e.g., a motor and a solenoid) for driving such components are connected to the control unit 16. An outdoor heat exchanger temperature sensor T1, an outdoor air temperature sensor T2, an indoor heat exchanger temperature sensor T3, and an indoor temperature sensor T4 are connected to the control unit 16. An irradiation unit 40 is connected to the control unit 16. Further, a filter cleaner 43 and a notification unit 45 are connected to the control unit 16.

The outdoor heat exchanger temperature sensor T1 is installed in the outdoor heat exchanger 8 to detect a temperature of the outdoor heat exchanger 8. The outdoor air temperature sensor T2 is installed in the outdoor unit 3 to detect an outdoor temperature. The indoor heat exchanger temperature sensor T3 is installed in the indoor heat exchanger 4 to detect a temperature of the indoor heat exchanger 4. The indoor temperature sensor T4 is installed in the indoor unit 2 to detect an indoor temperature.

The control unit 16 includes a microcomputer, an input-output circuit, and the like. The control unit 16 controls the operation of the air conditioner 1 by performing operation processing, determination processing, or the like based on a command (such as an operation start command or an indoor temperature setting command) sent from the remote control 17 or various temperatures detected by the outdoor heat exchanger temperature sensor T1, the outdoor air temperature sensor T2, the indoor heat exchanger temperature sensor T3, and the indoor temperature sensor T4.

### [Configuration of indoor unit]

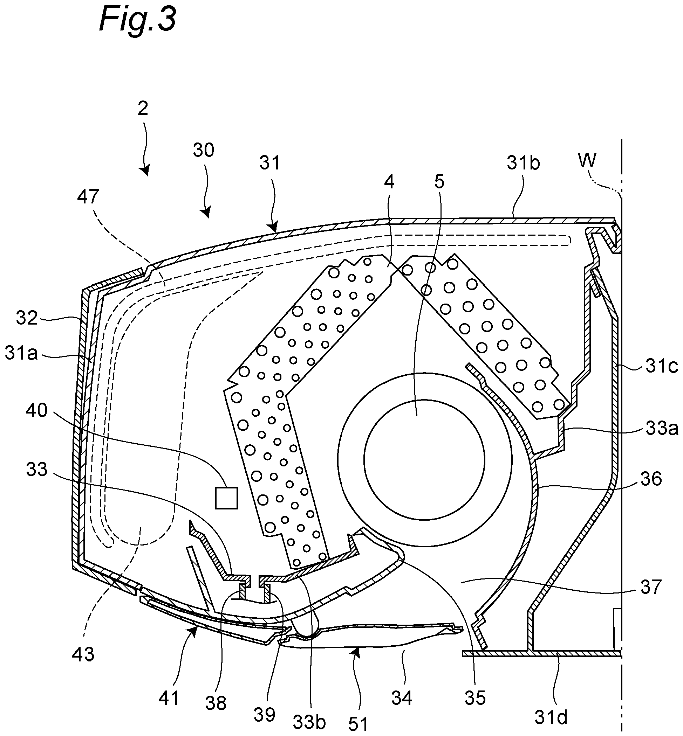

Fig. 3 is a schematic cross-sectional view of the indoor unit 2 that is out of operation, the indoor unit 2 being a component of the air conditioner 1. The indoor unit 2 illustrated in Fig. 3 is of a wall-mounted type.

The indoor unit 2 includes a casing 30 including a casing body 31 and a front panel 32. The casing 30 is attached to a wall surface W facing an indoor space and accommodates the indoor fan 5, the indoor heat exchanger 4, the drain pan 33, and the like.

The casing body 31 includes a plurality of parts: a front part 31a, an upper part 31b, a rear part 31c, and a lower part 31d. The front panel 32 is attached to the front part 31a in an openable and closable manner. Further, an intake port (not illustrated) is provided extending from the front part 31a to the upper part 31b.

The front panel 32 is associated with the front part 31a of the indoor unit 2 and has, for example, a flat shape with no intake port. Further, an upper end of the front panel 32 is pivotably supported by the upper part 31b of the casing body 31 and thus can swing in a hinged manner.

The indoor fan 5 and the indoor heat exchanger 4 are attached to the casing body 31. The indoor heat exchanger 4 exchanges heat with indoor air drawn into the casing 30 through the intake port. Further, the indoor heat exchanger 4 has an inverted V shape in a side view with both ends extending downward and a bend positioned higher. The indoor heat exchanger 4 includes a plurality of heat transfer tubes and a large number of fins.

The indoor fan 5 is positioned below the bend of the indoor heat exchanger 4. The indoor fan 5 is, for example, a cross-flow fan. The indoor fan 5 forces indoor air passing through the indoor heat exchanger 4 to flow to a blow-out port 34 of the lower part 31d of the casing body 31.

The casing body 31 is further provided with a first partition wall 35 and a second partition wall 36. A space between the first partition wall 35 and the second partition wall 36 serves as a blow-out flow path 37 through which the indoor fan 5 and the blow-out port 34 communicate with each other.

The drain pan 33 is disposed below the indoor heat exchanger 4 and receives condensed water generated by condensation on the indoor heat exchanger 4. The drain pan 33 includes an upper receiver 33a, a lower receiver 33b, and a connecting part (not illustrated) through which the upper receiver 33a and the lower receiver 33b are connected with each other. The condensed water drops from the indoor heat exchanger 4 into both the upper receiver 33a and the lower receiver 33b. The condensed water dropped into the upper receiver 33a flows down to the lower receiver 33b through the connecting portion. The condensed water flowing down from the upper receiver 33a to the lower receiver 33b and the condensed water dropped into the lower receiver 33b accumulate in the lower receiver 33b as drain water. The drain water accumulated in the lower receiver 33b is drained, by its own weight, outside from a drain port 38 provided in the lower receiver 33b through a drain hose 39. That is, the drain pan 33 is structured to cause the drain water to flow out byits own weight.

The control unit 16 controls the cooling operation to make the temperature of the indoor heat exchanger 4 measured by the indoor heat exchanger temperature sensor T3 lower than the dew point, thereby generating drain water. The control unit 16 can estimate a water level of the drain water accumulated in the lower receiver 33b of the drain pan 33 based on the operation status of the cooling operation. Therefore, the control unit 16 functions as a detection unit that detects the water level of the drain water accumulated in the drain pan 33. Some air conditioners, e.g., air conditioners installed at high places such as ceiling-embedded air conditioners and ceiling-suspended air conditioners, may have a water level sensor installed as a detection unit that detects the water level of the drain water accumulated in the drain pan 33.

The irradiation unit 40 (illustrated in Fig. 2 but not illustrated in Fig. 3) is provided above the drain pan 33. The irradiation unit 40 emits deep ultraviolet rays (hereinafter, referred to as "ultraviolet rays") having a relatively short wavelength among ultraviolet rays to irradiate an upper surface of the drain pan 33 with the ultraviolet rays. The irradiation unit 40 is, for example, an ultraviolet LED (light emitting diode). The ultraviolet rays emitted by the irradiation unit 40 have a wavelength of, for example, 255 nm to 350 nm.

In order to denature or inactivate bacteria, mold, or the like contained in the drain water, i.e., to perform sterilization, it is necessary to emit the ultraviolet rays by a predetermined dose. The dose of the ultraviolet rays to be emitted is determined by multiplying the ultraviolet intensity by the irradiation time, that is, by the ultraviolet intensity * the irradiation time. The control unit 16 controls the ultraviolet intensity and the irradiation time of the irradiation unit 40 so as to obtain the predetermined dose necessary for sterilization.

The irradiation unit 40 irradiates an irradiation area, i.e., an area to-be-irradiated such as the drain pan 33 with ultraviolet rays by the predetermined dose to sterilize the to-be-irradiated area, thereby allowing the inside of the indoor unit 2 to be kept clean.

The indoor unit 2 includes a first horizontal flap 41 and a second horizontal flap 51 disposed behind the first horizontal flap 41 (adjacent to the wall surface W). The first horizontal flap 41 and the second horizontal flap 51 adjust a vertical direction of air blowing out from the blow-out port 34 (air flowing through the blow-out flow path 37). The first horizontal flap 41 is pivotably attached to the lower part 31d of the casing body 31. In the state illustrated in Fig. 3, the indoor fan 5 is stopped, the front panel 32, the first horizontal flap 41, and the second horizontal flap 51 are closed, and the air conditioning operation by the indoor unit 2 is stopped. Note that the first horizontal flap 41 is an example of a first horizontal blade. Further, the second horizontal flap 51 is an example of a second horizontal blade.

The indoor unit 2 further includes a plurality of vertical flaps (not illustrated) that adjust a lateral direction of air blowing out. The plurality of vertical flaps are arranged in the blow-out flow path 37 at predetermined intervals in a longitudinal direction of the blow-out port 34 (a direction perpendicular to the drawing sheet of Fig. 3). Note that the vertical flap is an example of a perpendicular blade.

The indoor unit 2 is equipped with a filter 47 and the filter cleaner 43. The filter 47 is disposed to cover almost all over a frontside (air flow upstream side) of the indoor heat exchanger 4 so as to remove dust contained in the air flowing toward the indoor heat exchanger 4. The filter cleaner 43 is provided to clean the filter 47 to remove dust adhering to the filter 47. The filter cleaner 43 includes, for example, a rotary brush, a brush motor, a dust box, and a filter transfer means (none of which are illustrated). The dust removed from the filter 47 is accumulated in the dust box. Further, the dust box is detachably attached to the casing 30.

Further, the control unit 16 brings the rotary brush, the filter transfer means, and the like of the filter cleaner 43 into operation to control a filter cleaning operation. The filter cleaning operation thus performed can remove dust from the filter 47 accommodated in the casing 30.

The indoor unit 2 is further equipped with the notification unit 45. The notification unit 45 is a display device (for example, an LED or liquid crystal display device) or a speaker. The control unit 16 controls the display device to output a message about operation in a visual form. The control unit 16 controls the speaker to output the message about operation in an audio form.

For example, as will be described later, even when the user presses an "ON/OFF" button 66 of the remote control 17 to stop the irradiating operation, the irradiating operation is controlled to continue. At this time, the notification unit 45 notifies the user of the continuation of the irradiating operation in either the visual form or the audio form. The notification unit 45 can make the user aware of the continuation of the irradiating operation even when the user intends to stop the irradiating operation.

### [Remote control]

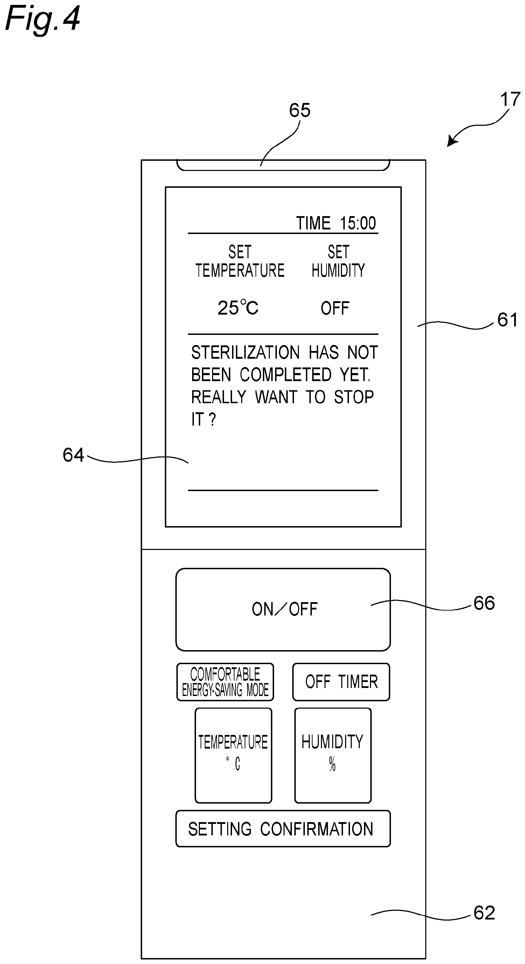

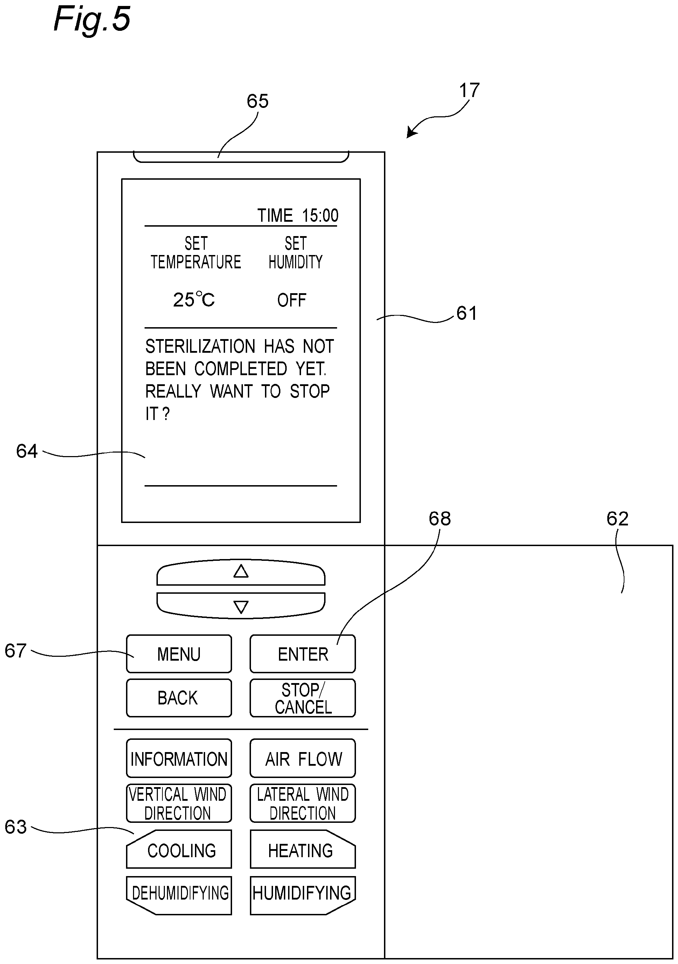

The remote control 17 is a device provided for remotely controlling the air conditioner 1, and includes, as illustrated in Figs. 4 and 5, a remote control casing 61, operation buttons, a display unit 64, and a remote control side control unit (not illustrated).

The remote control casing 61 has a substantially rectangular parallelepiped shape, and accommodates the remote control side control unit and the like. Further, as illustrated in Fig. 5, the remote control casing 61 includes a panel 62. The panel 62 is attached in an openable and closable manner to cover a front surface 63 of the remote control casing 61. Specifically, the panel 62 covers the front surface 63 of the remote control casing 61 when closed, and exposes the front surface 63 of the remote control casing 61 to the outside when opened.

The operation buttons are provided on the front surface 63 and the panel 62 of the remote control casing 61. As illustrated in Fig. 4, the panel 62 is provided with operation buttons such as the "ON/OFF" button 66, a "temperature" button, a "humidity" button, and a "setting confirmation" button. The "ON/OFF" button 66 receives, from the user, a request for the start or stop of the normal operation and a second maintenance operation of the air conditioner 1. As described later, the second maintenance operation is an operation obtained by removing an ultraviolet rays irradiating operation serving as a first maintenance operation from the maintenance operation, and includes, for example, the filter cleaning operation and an internal cleaning operation.

As illustrated in Fig. 5, various operation buttons such as a "menu" button 67, an "enter" button 68, and an "up" or "down" button are provided on the front surface 63 of the remote control casing 61. The "menu" button 67 and the "enter" button 68 are buttons to be operated by the user when the user changes an operating mode or function setting.

The "menu" button 67 acts as a forced operation unit to forcibly stop the ultraviolet rays irradiating operation. The "menu" button 67 is structured such that the menu has a hierarchy. A setting menu used to enable a forced operation to forcibly stop the irradiating operation is provided as a lower level in the hierarchical structure of the "menu" button 67. When the user presses the "menu" button 67 a plurality of times, a setting key used to enable the forced operation to forcibly stop the irradiating operation is displayed on the display unit 64. When the user presses the "up" or "down" button to select the setting key associated with the forced operation and then presses the "enter" button 68, a signal associated with the forced operation is transmitted to the indoor unit 2. As described above, such a plurality of hierarchical operations, which make a forced operation complicated, are required to enable the forced operation to forcibly stop the irradiating operation, so that it is possible to prevent the user from stopping the irradiating operation. The forced operation enabled by the forced operation unit (pressing the "menu" button 67 in a hierarchical manner) is different from the stop operation (pressing the "ON/OFF" button 66) performed to stop the air conditioning operation and the stop operation performed to stop the second maintenance operation. Since the forced operation is different from the stop operation to stop the air conditioning operation and the stop operation to stop the maintenance operation, it is possible to prevent the user from stopping the irradiating operation.

The display unit 64 may be any type of display device, and is, for example, a liquid crystal display device. The control unit 16 controls the display unit 64 to display details of information, message, or the like transmitted from the air conditioner 1. The display unit 64 of the remote control 17 acts as a notification unit and displays various messages about operation. For example, a warning message is displayed on the display unit 64 when the forced operation is performed on the ultraviolet rays irradiating operation.

In some embodiments, the remote control 17 may be further equipped with a speaker that acts as the notification unit. The control unit 16 controls the speaker to output various messages about operation in an audio form.

According to the present embodiment, an operation unit used to start or stop the normal operation of the air conditioner 1 and to start or stop the second maintenance operation of the air conditioner 1 is provided as the "ON/OFF" button 66 of the remote control 17. In some embodiments, the operation unit may be provided in the indoor unit 2.

Next, the normal operation and maintenance operation of the air conditioner 1 will be described.

### [Normal operation and maintenance operation]

### (1) Normal operation

The normal operation includes the cooling operation, the dehumidifying operation, and the heating operation. During the normal operation, the compressor 6 is driven, and the opening of the expansion valve 10 is reduced to a predetermined degree, thereby causing the refrigerant to circulate through the refrigerant circuit and causing the indoor heat exchanger 4 to function as a condenser or an evaporator. Further, when the indoor fan 5 is driven, air is drawn into the indoor unit 2 through the intake port, flows through the indoor heat exchanger 4, and then blows out from the blow-out port 34 into the room. This allows the room to be cooled, dehumidified, or heated.

### (2) Maintenance operation

The maintenance operation is performed to keep the indoor heat exchanger 4, the filter 47, and the drain pan 33, which are components built in the indoor unit 2, clean. The maintenance operation includes, for example, the internal cleaning operation, the filter cleaning operation, and the ultraviolet rays irradiating operation, and any operation to keep the components built in the indoor unit 2 clean may belong to the maintenance operation. For example, assuming that the ultraviolet rays irradiating operation belongs to the first maintenance operation, the internal cleaning operation, the filter cleaning operation, and the other cleaning operation belong to the second maintenance operation.

The internal cleaning operation is provided to suppress propagation of bacteria, mold, or the like in the indoor unit 2. During the internal cleaning operation, a blowing operation and heating operation are performed for a predetermined time after performing the cooling operation or the dehumidifying operation to dry the indoor heat exchanger 4. This allows the inside of the indoor unit 2 to be kept clean.

The filter cleaning operation is provided to automatically clean the filter 47. During the filter cleaning operation, the filter cleaner 43 is brought into operation to clean the filter 47. This makes it possible to automatically remove dust adhering to the filter 47 to keep the inside of the indoor unit 2 clean.

The ultraviolet rays irradiating operation is provided to suppress propagation of bacteria, mold, or the like in the indoor unit 2. During the ultraviolet rays irradiating operation, after the cooling operation or the dehumidifying operation, an irradiation area such as the indoor heat exchanger 4, the filter 47, or the drain pan 33 where bacteria, mold, or the like easily propagates is irradiated with the ultraviolet rays for a predetermined time. This makes it possible to sterilize the irradiation area to keep the inside of the indoor unit 2 clean.

The control unit 16 causes the maintenance operation to be automatically activated after the air conditioning operation is stopped. The control unit 16 determines that the maintenance operation is necessary when an accumulated time during which the air conditioning operation has been performed is equal to or longer than a predetermined time, and causes the maintenance operation to be automatically activated after the air conditioning operation is stopped. Alternatively, the control unit 16 causes the maintenance operation to be activated at a predetermined timing set as necessary by the user and after the air conditioning operation is stopped.

### [Control of maintenance operation]

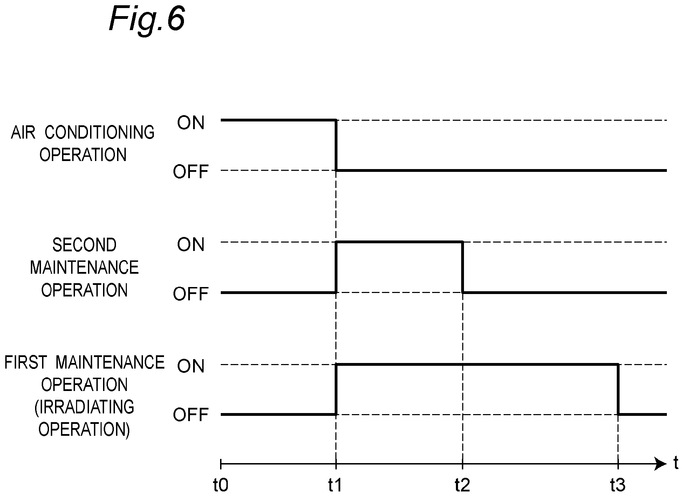

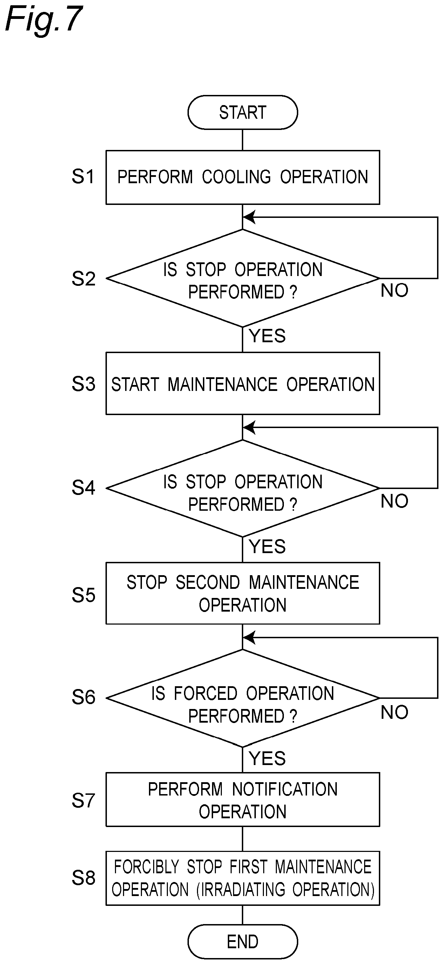

Next, the control of the maintenance operation of the air conditioner 1 will be described with reference to Figs. 6 and 7. Fig. 6 is a timing chart of the maintenance operation of the air conditioner 1. Fig. 7 is a control flowchart of the maintenance operation of the air conditioner 1.

In the air conditioner 1, when the cooling operation is selected by operation of the remote control 17 performed by the user, the control unit 16 performs the cooling operation desired by the user at an operation start time t0 shown in Fig. 6 to place the air conditioner 1 in the cooling operation over a predetermined period of time (step S1).

In step S2, for example, the control unit 16 determines whether the stop operation of pressing the "ON/OFF" button 66 has been performed. When the stop operation has not been performed (NO in step S2), the process waits until the stop operation is performed. When the stop operation has been performed (YES in step S2), the process proceeds to step S3.

In step S3, the control unit 16 stops the cooling operation and performs the maintenance operation at a stop operation time t1 shown in Fig. 6. Specifically, the control unit 16 performs at least either the internal cleaning operation or the filter cleaning operation as the second maintenance operation, and the ultraviolet rays irradiating operation as the first maintenance operation. As a result, both the second maintenance operation and the first maintenance operation (ultraviolet rays irradiating operation) are performed.

In step S4, for example, the control unit 16 determines whether the stop operation of pressing the "ON/OFF" button 66 has been performed. When the stop operation has not been performed (NO in step S4), the process waits until the stop operation is performed. When the stop operation has been performed (YES in step S4), the process proceeds to step S5.

In step S5, the control unit 16 stops the internal cleaning operation or the filter cleaning operation as the second maintenance operation and continues the ultraviolet rays irradiating operation as the first maintenance operation at a stop time t2 shown in Fig. 6. Stopping the second maintenance operation in response to the stop operation on the second maintenance operation performed by the user makes it possible to reflect the intention of the user to stop the second maintenance operation.

In step S6, the control unit 16 determines whether the forced operation of pressing the "menu" button 67 a plurality of times, pressing the "up" or "down" button, and pressing the "enter" button 68 has been performed. When the forced operation has not been performed (NO in step S6), the process waits until the forced operation is performed. When the forced operation has been performed (YES in step S6), the process proceeds to step S7.

In step S7, the control unit 16 controls the display unit 64 to perform a notification operation. As described above, the display unit 64 acts as the notification unit to output a warning message in a visual form when the forced operation is performed on the ultraviolet rays irradiating operation. The display unit 64 displays, for example, "Sterilization has not been completed yet. Really want to stop it?" as the warning message when the forced operation is performed on the ultraviolet rays irradiating operation. Alternatively, the speaker (notification unit 45) provided in the indoor unit 2 or the speaker (not illustrated) provided in the remote control 17 can output a similar warning message in an audio form. This notification can make the user aware that the stop of the irradiating operation is not preferable for sterilization

In step S8, the control unit 16 controls the irradiation unit 40 to forcibly stop the ultraviolet rays irradiating operation at a forced stop time t3 shown in Fig. 6. Specifically, the control unit 16 controls the irradiation unit 40 to turn off the irradiation unit 40. In step S7, it is possible to reflect the intention of the user by making the user aware that the stop of the irradiating operation is not preferable for sterilization t and then stopping the irradiating operation. When the irradiation unit 40 is turned off, the control of the maintenance operation is brought to an end.

In the air conditioner 1, the irradiating operation continues even when the stop operation to stop the maintenance operation is performed, thereby allowing the inside of the indoor unit 2 to be kept clean.

The embodiment of the present invention has been described above. However, it should be understood that specific configurations of the present invention are not limited to those described in the embodiment. The scope of present invention is indicated by not only the embodiment described above but also the appended claims and further includes all modifications within the scope of the claims.

Note that an operation unit used to stop the second maintenance operation of the maintenance operation and an operation unit used to stop the air conditioning operation may be separately provided.

Note that the second maintenance operation includes an internal cleaning operation or a filter cleaning operation or both of these cleaning operations.

### REFERENCE SIGNS LIST

- 1: air conditioner
- 2: indoor unit
- 3: outdoor unit
- 4: indoor heat exchanger (heat exchanger)
- 6: compressor
- 7: four-way switching valve
- 8: outdoor heat exchanger (heat exchanger)
- 10: expansion valve
- 11: accumulator
- 14: indoor-unit controller (control unit)
- 15: outdoor-unit controller (control unit)
- 16: control unit
- 17: remote controller (remote control)
- 30: casing
- 31: casing body
- 31a: front part
- 31b: upper part
- 31c: rear part
- 31d: lower part
- 32: front panel
- 33: drain pan (an irradiation area to-be-irradiated )
- 34: blow-out port
- 35: first partition wall
- 36: second partition wall
- 37: blow-out flow path
- 38: drain port
- 39: drain hose
- 40: irradiation unit
- 41: first horizontal flap
- 43: filter cleaner
- 45: notification unit
- 47: filter
- 51: second horizontal flap
- 61: remote control casing
- 62: panel
- 63: front surface
- 64: display unit (notification unit)
- 65: transmitter
- 66: "ON/OFF" button (operation unit)
- 67: "menu" button (forced operation unit)
- 68: "enter" button
- L1, L2: connection pipe
- T1: outdoor heat exchanger temperature sensor
- T2: outdoor air temperature sensor
- T3: indoor heat exchanger temperature sensor
- T4: indoor temperature sensor
- t0: operation start time
- t1: stop operation time
- t2: stop time
- t3: forced stop time
- W: wall surface

## Claims

1. An air conditioner (1) comprising:
a control unit (16) configured to control an air conditioning operation and control a maintenance operation to perform maintenance on an indoor unit (2) of the air conditioner (1) after the air conditioning operation is stopped; and
an operation unit (66) provided for performing a stop operation to stop the maintenance operation, wherein
the maintenance operation includes an irradiating operation to irradiate an irradiation area (33) of the indoor unit (2) with ultraviolet rays, and
the control unit (16) controls the irradiating operation to continue the irradiating operation even when the stop operation is performed by the operation unit (66),
**characterized in that**:
the maintenance operation includes a second maintenance operation including at least either an internal cleaning operation or a filter cleaning operation, and
wherein, when the stop operation is performed, the control unit (16) controls the second maintenance operation to stop the second maintenance operation.

2. The air conditioner (1) according to claim 1, further comprising a notification unit (45) configured to issue a notification about the irradiating operation, wherein the control unit (16) controls the notification unit (45) to issue the notification about the continuation of the irradiating operation.

3. The air conditioner (1) according to claim 2, wherein when a forced operation is performed to forcibly stop the irradiating operation, the control unit (16) controls the notification unit (45) to issue the notification about the stop of the irradiating operation and controls the irradiating operation to forcibly stop the irradiating operation.

4. The air conditioner (1) according to claim 3, wherein the forced operation is an operation different from the stop operation to stop the maintenance operation.

## Patentansprüche

1. Klimaanlage (1), umfassend
eine Steuereinheit (16), die konfiguriert ist zum Steuern eines Klimatisierungsvorgangs und zum Steuern eines Wartungsvorgangs, um nach Beendigung des Klimatisierungsvorgangs eine Wartung an einer Inneneinheit (2) der Klimaanlage (1) durchzuführen; und
eine Betriebseinheit (66), die zur Durchführung eines Beendigungsvorgangs vorgesehen ist, um den Wartungsvorgang zu beenden, wobei
der Wartungsvorgang einen Bestrahlungsvorgang einschließt, um einen Bestrahlungsbereich (33) der Inneneinheit (2) mit ultravioletten Strahlen zu bestrahlen, und
die Steuereinheit (16) den Bestrahlungsvorgang steuert, um den Bestrahlungsvorgang fortzusetzen, selbst wenn der Beendigungsvorgang von der Betriebseinheit (66) durchgeführt wird,
**dadurch gekennzeichnet, dass**:
der Wartungsvorgang einen zweiten Wartungsvorgang einschließt, der zumindest entweder einen Innenreinigungsvorgang oder einen Filterreinigungsvorgang einschließt, und
wobei, wenn der Beendigungsvorgang durchgeführt wird, die Steuereinheit (16) den zweiten Wartungsvorgang steuert, um den zweiten Wartungsvorgang zu beenden.

2. Klimaanlage (1) nach Anspruch 1 weiter umfassend eine Benachrichtigungseinheit (45), die konfiguriert ist zum Herausgeben einer Benachrichtigung über den Bestrahlungsvorgang, wobei die Steuereinheit (16) die Benachrichtigungseinheit (45) steuert, um die Benachrichtigung über die Fortsetzung des Bestrahlungsvorgangs herauszugeben.

3. Klimaanlage (1) nach Anspruch 2, wobei, wenn ein erzwungener Vorgang durchgeführt wird, um den Bestrahlungsvorgang zwangsweise zu beenden, die Steuereinheit (16) die Benachrichtigungseinheit (45) steuert, um die Benachrichtigung über die Beendigung des Bestrahlungsvorgangs herauszugeben, und den Bestrahlungsvorgang steuert, um den Bestrahlungsvorgang zwangsweise zu beenden.

4. Klimaanlage (1) nach Anspruch 3, wobei der erzwungene Vorgang ein anderer Vorgang ist als der Beendigungsvorgang zum Beendigen des Wartungsvorgangs.

## Revendications

1. Climatiseur (1), comprenant :
une unité de commande (16) configurée pour commander une opération de climatisation et commander une opération de maintenance pour mettre en oeuvre une maintenance sur une unité intérieure (2) du climatiseur (1) après l'arrêt de l'opération de climatisation ; et
une unité d'actionnement (66) prévue pour mettre en oeuvre une opération d'arrêt afin d‴arrêter l'opération de maintenance, dans lequel
l'opération de maintenance inclut une opération d'irradiation pour irradier une zone d'irradiation (33) de l'unité intérieure (2) avec des rayons ultraviolets, et
l'unité de commande (16) commande l'opération d'irradiation pour poursuivre l'opération d'irradiation même lorsque l'opération d'arrêt est mise en oeuvre par l'unité d'actionnement (66),
**caractérisé en ce que** :
l'opération de maintenance inclut une seconde opération de maintenance incluant au moins soit une opération de nettoyage interne, soit une opération de nettoyage de filtre, et
dans lequel, lorsque l'opération d'arrêt est mise en oeuvre, l'unité de commande (16) commande la seconde opération de maintenance pour arrêter la seconde opération de maintenance.

2. Climatiseur (1) selon la revendication 1, comprenant en outre une unité de notification (45) configurée pour délivrer une notification concernant l'opération d'irradiation, dans lequel l'unité de commande (16) commande l'unité de notification (45) pour délivrer la notification concernant la poursuite de l'opération d'irradiation.

3. Climatiseur (1) selon la revendication 2, dans lequel lorsqu'une opération forcée est mise en oeuvre pour arrêter de force l'opération d'irradiation, l'unité de commande (16) commande l'unité de notification (45) pour délivrer la notification concernant l'arrêt de l'opération d'irradiation et commande l'opération d'irradiation pour arrêter de force l'opération d'irradiation.

4. Climatiseur (1) selon la revendication 3, dans lequel l'opération forcée est une opération différente de l'opération d'arrêt pour arrêter l'opération de maintenance.
